Europäisches Patentamt

(19) European Patent Office

Office européen des brevets

(11) Publication number: **0 076 647**
**A2**

(12) # EUROPEAN PATENT APPLICATION

(21) Application number: **82305187.5**

(22) Date of filing: **30.09.82**

(51) Int. Cl.³: **C 12 N 5/00**

(30) Priority: **02.10.81 US 307871**

(43) Date of publication of application:
**13.04.83 Bulletin 83/15**

(84) Designated Contracting States:
**AT BE CH DE FR GB IT LI LU NL SE**

(71) Applicant: HANA Biologics Incorporated
1400 53rd Street Suite M443
Emeryville California 94608(US)

(72) Inventor: Steimer, Kathelyn Sue
2450 Warrning Apt.24
Berkeley California 94704(US)

(74) Representative: SERJEANTS
25, The Crescent
Leicester, LE1 6RX(GB)

(54) Culture media for cells originating from the immune system.

(57) A culture medium, useful for the serum-free culturing of a variety of cells originating from the immune system and for culturing cells with a serum supplement, is disclosed. The cells to be cultured include, in addition to hybridomas, other cell types such as a variety of malignant cells of the immune system and normal lymphocytes.

EP 0 076 647 A2

-1-

TITLE:

Culture Media for Cells Originating from the Immune
System

DESCRIPTION:

FIELD OF THE INVENTION

The invention relates to culture media, and in
particular to media for culturing cells originating
from the immune system in serum-free or low serum
conditions.

BACKGROUND OF THE INVENTION

In 1975, Kohler and Milstein reported that it was
possible to immortalize normal antibody-producing cells
by fusing them with myeloma cells (Kohler, G. and C.
Milstein. 1975, Nature 256:495). The resulting
hybrids, known as hybridomas, produce homogenous
or monoclonal antibodies specific for a single
antigenetic determinant. The use of monoclonal
antibodies has become common in almost every area of
biology. For example, monoclonal antibodies have been used to study cell surface antigens, for affinity purification of proteins, for histocompatibility testing, for studying various viruses and for radioimmunoassay. More recently, it has been recognized that monoclonal antibodies may have medical application such as for drug targeting, immunotherapy and as reagents for absorbing toxic substances.

It is often desirable, and in some cases essential, to purify the monoclonal antibodies produced by hybridoma cell lines. In vitro, hybridomas are usually grown in nutrient medium supplemented with ten to twenty percent serum. Since antibody production by hybridomas is usually in the range of one to one hundred micrograms of immunoglobulin protein per millilitre of culture fluid, the use of serum introduces a vast excess of heterologous protein that must be eventually removed during antibody purification. The development of a serum-free medium for culturing hybridoma cell lines would greatly simplify the task of antibody purification.

There has been some progress in attempting to grow mouse hybridoma cell lines in serum-free media. For example, complete Iscove's modified Eagles medium, which is not commercially available, was developed for the serum-free culturing of murine lymphocytes and has been used with some success for growing hybridomas derived from FO (Fazekas de St. Groth, S. and D. Schiedegger. 1980. J. Immunol. Methods 35:1), a variant of the parental myeloma line SP2/0-Ag-14 (Schulman, M. et al. 1978. Nature 276:269). However the difficulties in preparing complete Iscove's medium make it a less than desirable alternative to serum containg media. It has further been reported that P3-NS-1-Ag-4-1 hybridoma cell lines (Chang, T. et al., 1980. J. Immunol. Methods 39:369), but not the parental myeloma, will grow in a very simple medium consisting of enriched RPMI 1640 supplemented with only insulin and transferrin. However, the generation times of these hybridomas are on the order of three days in the serum-free medium as compared to less than one day in serum-supplemented medium.

The present invention provides a culture medium without the drawbacks of the prior art. This medium may be used for the serum-free culturing of a variety of cells originating from the immune system. This includes, in addition to hybridomas, other cell types such as a variety of malignant cells of the immune system and normal lymphocytes. The present invention also provides a culture medium that improves the growth of the above cell types when used with a serum supplement.

## SUMMARY OF THE INVENTION

A culture medium according to the invention has the composition set out in the characterising part of claim 1. This medium is suitable for growing myeloma and hybridoma cell lines, particularly those of murine origin in a serum-free environment.

## DETAILED DESCRIPTION OF THE INVENTION

As adjusted to concentrations that optimize growth, the culture medium according to the invention has the composition set out in the characterizing part of claim 2.

The balanced salt solution most preferably comprises the following salts in the following concentrations:

approximately 100 mg/l $Ca(NO_3)_2 \cdot 4H_2O$

approximately 400 mg/l KCl

approximately 100 mg/l $MgSO_4 \cdot 7H_2O$

approximately 6000 mg/l NaCl; and

approximately 800 mg/l $Na_2HPO_4$.

The above balanced salt solution is known as Earle's balanced salt solution, as taught by Bryant in Preparation of Saline, in Tissue Culture Association Manual, Vol. 1, No. 4, pp. 185-187 (1975). However, any balanced salt solution may be used. One possible alternative is Hank's balanced salt solution as taught by Hank's & Wallace in <u>Relation of Oxygen and Temperature in Preservation of Tissues by Refrigeration</u>, Proc. Soc. Exp. Biol. Med., 71:196-200 and by Hank's in <u>Hank's Balanced Salt Solution and pH Control</u>, Tissue Culture Association Manual, Vol. 1, No. 1, pp. 3-4 (1975).

These formulations provide a means of avoiding the use of serum in the <u>in vitro</u> culture of hybridoma cell lines for the purpose of producing and eventually purifying monoclonal antibodies. Serum is a complex biological fluid containing hundreds of different proteins. The use of serum for hybridoma cell culture introduces a vast excess of protein (approximately 6-30 mg/ml) to the growth medium that must be removed during monoclonal antibody purification. By contrast, the medium of the present invention contains only three proteins, all purified, and their total maximum possible concentration is only approximately 765 micrograms/ml. Monoclonal antibodies may thus be purified from the defined medium supernatants of the present invention by simple protein fractionization procedures rather than by the more sophisticated techniques such as affinity chromatography that are essential when hybridomas are grown in serum supplemented medium.

The medium described herein can be used for growing

SP2/0-Ag-14 mouse myeloma cells and hybridomas derived from this parent, certain P3-NS1-Ag-4-1 hybridomas, FO mouse myeloma and hybridoma cell lines, and MPC-11 myeloma and hybridoma cell lines. It may also be used for the serum-free culturing of human lymphoblastoid cell lines and human hybridoma cell lines, and as a culture medium for normal lymphocytes. It has been effective for both mitogen and antigen stimulation of proliferation and antibody production by mixed lymphocyte cultures. Since a variety of cells of the immune system can be cultured in this medium, it may facilitate the purification of effector molecules such as lymphokines or other growth factors produced in vitro by malignant, transformed, and/or normal cells of the immune system either by producing a culture system for obtaining such products in vitro in the absence of added serum or by providing a means for devising an assay system for such effector molecules. For example, the serum-free growth of certain cells may depend upon or be stimulated by such effector molecules.

It was first discovered that a very simple combination of hormones and growth factors would support the serum-free growth of SP2/0-Ag-14 mouse myeloma cells when added to a suitable nutrient medium. It was determined that the optimum supplement consisted of insulin, transferrin, ethanolamine and bovine serum albumin (BSA) complexed with essential and non-essential fatty acids. The nutrient medium described in this application was optimized for growing SP2/0-Ag-14 mouse myeloma cells with the above described growth factors. The optimal recipe for growing SP2/0-Ag-14 was then tested on a variety of different cell types (described below as examples). This formulation proved to be effective for a variety of different cell culture applications.

Preparation of Medium

The serum-free culture medium of the present invention can be routinely prepared by the simple addition of each ingredient recited above. Generally, insulin, transferrin, fatty acid linked BSA, antibiotics, glutamine and sodium pyruvate are added immediately before using the medium. The nutrient medium (without the above listed ingredients) is prepared and stored as a liquid at 4-8°C for no longer than 4 months. The basal medium may also be prepared as a dried powder in a manner similar to other conventional tissue culture media, reconstituted with distilled water and filter sterilized. Stocks of insulin in 0.1 N HCl are prepared at 100-1000 fold higher concentration than in the final recipe and stored at 4°C as a liquid. A 50X stock of transferrin, ethanolamine, and fatty acid linked BSA are prepared and stored frozen.

The bovine serum albumin, sold under the tradename Pentex by Miles Biochemical of Elkhardt, Indiana, is linked in a 1:1 molar ratio with one or more of the following fatty acids: linoleic acid, linolenic acid, palmitoleic acid, oleic acid, arachadonic acid, palmitic acid and stearic acid. More specifically, a solution of $1.5 \times 1^{-2}$M fatty acid (0.5 ml) in 95% ethanol is added dropwise, while mixing to 10 ml of bovine serum albumin (50 mg/ml) in phosphate buffered saline). Stocks of each fatty acid complexed individually with bovine serum albumin are prepared, filter sterilized, and stored at -20°C. Any suitable BSA preparation may be substituted for the Pentex BSA and the ratio of BSA to fatty acid may be in the range of approximately 0.5-4.0 and still support near optimal growth.

## Cell Growth Assays

The experimental protocol for testing the growth of a particular cell line in the medium of the present invention was as follows. The cells were detached from their culture flasks by gently pipetting the culture medium over the growth surface or, if the cells were growing in suspension, the cells were gently pipetted to ensure a single cell suspension. The resuspended cells were centrifuged (for example, 800 xg) in a clinical centrifuge, washed once with phosphate buffered saline, and resuspended in phosphate buffered saline. Aliquots of cell suspension were added to the medium of the present invention to yield a final cell density of $2 \times 10^4$ cells/millilitre. Control media tested in parallel were unsupplemented basal medium and basal medium supplemented with 10% fetal bovine serum, basal medium being the present invention without insulin, transferrin, ethanolamine, and fatty acid complexed with BSA. Aliquots of cell suspension were added to 24 well microtiter plates, each well receiving 1 ml of cell suspension. The plates were incubated at approximately 37°C in approximately a 7% $CO_2$-93% air atmosphere. Cultures were monitored daily microscopically and counted after 3 to 5 days. Cell counts were made by using a Coulter electronic particle counter.

Cells were serially subcultured in 5 ml of growth medium in 25 $cm^2$ plastic tissue culture flasks every 3-4 days. The cells were diluted to $2 \times 10^4$ cells/ml at each passage. On first passage, the cells were prepared as described above. For later passages, the cells were resuspended and diluted directly into their respective growth media.

For cell counting, cultures in 24 well microtiter plates were detached from the growth surface by vigorous pipetting. The resulting cell suspensions were diluted to 10 ml with Isoton II (Counter Diagnostics) and cell counts were determined using a Coulter particle counter (Model Zf, Coulter Electronics, Hialeah, Florida). Cultures growing in flasks (25 cm$^2$) were resuspended and either 0.2 ml or 1.0 ml of suspension diluted and counted.

Assay for Antibody Production

All of the hybridoma cell lines described in the examples belo were of murine origin and all produced antibody with specificity for keyhole limpet hemocyanin. The culture supernatants from hybridoma cell lines were collected and assayed for levels of IgG antibody with specificity for keyhole limpet hemocyanin by an indirect enzyme linked immunoassay [ELISA; Voller, A., D. Bidwell and A. Bartlett (1979). The Enzyme Linked Immunosorbent Assay (ELISA): A guide with abstracts of microplate applications. Sponsored and available from Dynatech Europe, Borough House, Rue du Pre, Guernsey, Great Britain]. Polystyrene culture tubes were coated with antigen by adding 0.5 ml of keyhole limpet hemocyanin (20 g/ml) in a 0.1 M carbonate-bicarbonate buffer (pH 9.6). The tubes were incubated at 4°C overnight. At the end of the incubation period, the tubes were washed three times with phosphate buffered saline containing 0.05% Tween 20 and 0.02% sodium azide (PBS-tween), shaken dry, and 0.5 ml of diluted culture supernatant added to each tube. Each culture supernatant was diluted 1/10 and by serial two-fold dilutions thereafter. Dilutions were assayed in duplicate using antigen coated tubes and singly using uncoated tubes. The tubes containing diluted supernatants were incubated at 37°C for two hours. At the end of the incubation period, the tubes were washed three times with PBS-tween, shaken dry and 0.5 ml of alkaline phosphatase conjugated anti-mouse IgG, made in rabbit (Miles-Yeda, Elkhardt, Indiana, cat §61-278) diluted inPBS-tween as recommended by the supplier, was added to each tube. The tubes were incubated for one hour at 37°C, washed three times with pBS-tween and 0.5 ml of 1mg/ml p-nitrophenyl phosphate in diethanolamine buffer (10% diethanolamine, 0.02% $NaN_3$, 0.01% $MgCl_2.6H_2O$, pH 9.8) was added to each tube. After 30 minutes incubation at 37°C, 0.25 ml of 3N NaOH was added to each tube. The absorbance of each reaction mixture was determined at 405 nm. Dilutions yielding $OD_{405}$ readings of 0.4 to 0.7 were used for calculating the reported values. The $OD_{405}$ readings of uncoated tubes and negative controls were always less than 0.03 units. The data is expressed as absorbance units per $10^4$ cells.

Generation of Hybridomas

In order to evaluate the effectiveness of the medium of the present invention as a culture medium for supporting the growth of and antibody production by murine hybridoma cultures, it was necessary to generate such hybridomas. Balb/c mice were immunized with keyhole limpet hemocyanin, their spleens removed and fused with either SP2/0-Ag-14 mouse myeloma or P3-NS-1-Ag-4-1 mouse myeloma cells. The fusing agent was polyethylene glycol 1500. The methods for cell fusion and hybridoma cell culture are described by Galfre, G., S. How, C. Milstein, G. Butcher, and J. Howard, 1977. Nature $\underline{266}$:550.

## EXAMPLE I

SP2/0-Ag-14 mouse myeloma cells were cultured in medium of the present invention and basal medium supplemented with 10% fetal bovine serum. Cells were plated at $2 \times 10^4$ cells per well in 24 well microtiter dishes and cell counts made daily. The generation time of SP2/0-Ag-14 was approximately 18 hours in the above defined medium and 15.5 hours in basal nutrient medium supplemented with serum SP2/0-Ag-14 cells could be repeatedly subcultured in the medium of the present invention. The generation time of the SP2/0-Ag-14 cells after 12 passages in this serum-free medium was approximately 17 hours, essentially the same as the generation time during the first passage in the medium. It was noted that the SP2/0-Ag-14 cells tended to grow in a suspension as single cells in the serum-free medium rather than loosely attached to the culture flask as they normally do in serum.

The importance of insuline, transferrin, ethanolamine, and fatty acid complexed BSA was determined by preparing medium lacking the specific component. It was found that the omission of insulin decreased the final cell density to 65% of that observed in using the above recited formulary of the present invention. Transferrin was absolutely essential for growing SP2/O-Ag-14 cells as without it, there was no growth. The omission of ethanolamine did not significantly affect cell growth while the absence of fatty acid-linked BSA components resulted in cell growth less than 10% of that attained through the use of the entire composition. It was also noted that when a fatty acid free BSA was added instead of fatty acid-linked BSA, the SP2/O-Ag-14 final cell density was less than 5% of that observed in the complete composition.

## EXAMPLE II

As stated previously, the medium of the present invention developed for culturing SP2/O-Ag-14 mouse myeloma cells also supports the growth of SP2/O-Ag-14 hybridomas. SP2/O-Ag-14 cells and four established SP2/O-Ag-14 hybridoma cell lines producing antibodies to keyhole limpet hemocyanin were cultured in the above described formulary and in basal nutrient medium supplemented with 10% fetal bovine serum. After approximately four days in culture, the SP2/O-Ag-14 cells reached a density in the medium of the present invention that was approximately 85% of that in serum supplemented basal medium. The cell density of the four hybridoma cell lines in the medium of the present invention ranged from 24 to 75% of that attained in serum supplemented basal medium as shown in Table I.

Table I. Growth of SP2/O-Ag-14 mouse hybridoma cell lines in serum-free medium.[a]

| | | Cells/ml in | |
| | | Serum-Free Medium[b] | Serum-Supplemented Medium[c] |
| Cell Line | Description | | |
|---|---|---|---|
| SP2/O-Ag-14 | Parental myeloma | $1.1 \times 10^6$ | $1.3 \times 10^6$ |
| S8-6-G1(D11) | SP2/O-Ag-14 hybridoma[d] | $6.7 \times 10^5$ | $1.3 \times 10^6$ |
| S1-1-D4(E2) | " | $5.8 \times 10^5$ | $7.1 \times 10^5$ |
| S8-2-D12(F5) | " | $2.6 \times 10^5$ | $1.1 \times 10^6$ |
| S4-3-DF(D1) | " | $7.1 \times 10^5$ | $1.1 \times 10^6$ |

[a] Cells of each type ($2 \times 10^4$/ml) were plated in 24 well microtiter plates in either serum-free or serum-supplemented medium. Cell counts were made four days after plating.

[b] Medium of the present invention.

[c] Basal nutrient medium with 10% fetal bovine serum.

[d] All hybridomas produce IgG antibodies with specificity for keyhole limpet hemocyanin.

Although the densities reached by the hybridomas were significantly lower in the medium of the present . invention as compared to serum supplemented medium, the increase in cell number over the initial plating value was substantial in each case. For example, the cell density in the medium of the present invention, of the hybridoma cells that grew the slowest of the four cells tested, /S8-2-D12 (F5)7 experienced a thirteen fold increase over their initial plating value. The antibody titers in culture supernatants from all four hybridoma cell lines grown in the serum-free medium were equal to or greater than the antibody titers in serum-supplemented medium as shown in Table II.

Table II. Titration of antibody production by hybridomas grown in serum-free and serum-supplemented media.[a]

|  |  | Antibody Production ($OD_{405}/10^4$ cells) | |
| --- | --- | --- | --- |
| Cell Line | Description | Serum-Free[b] | Serum-Supplemented[c] |
| SP2/O-Ag-14 | Parental Myeloma | 0.0002 | 0.0001 |
| S8-6-G1(D11) | SP2/O-Ag-14 hybridomas | 16.6 | 14.6 |
| S1-1-D4(E2) | " | 8.8 | 5.8 |
| S8-2-D12(FS) | " | 31.1 | 23.6 |
| S4-3-4F(D1) | " | 16.1 | 18.7 |

[a]Each cell line was plated ($2x10^4$/ml) in either serum-free or serum-supplemented medium. After four days the supernatants were collected and assayed for antibody with specificity for keyhole limpet hemocyanin by the quantitative ELISA assay. Cell counts were performed at the time that the supernatants were collected.

[b]Medium of the present invention.

[c]Basal nutrient medium supplemented with 10% fetal bovine serum.

[d]These hybridomas are producing monoclonal antibodies with specificity for keyhole limpet hemocyanin.

As mentioned in Example I, SP2/0-Ag-14 myeloma cells grew in suspension in the medium of the present invention. All four SP2/0-Ag-14 hybridomas also grew in suspension in this medium.

All of the hybridoma cell lines were serially subcultured in the medium of the present invention for at least seven passages representing approximately 30 to 35 population doublings. One hybridoma cell line S1-1-D4(E2) was serially subcultured, the growth rate examined periodically, and culture supernatants harvested to be assayed for antibody titers. The generation times in passage 1 in the medium of the present invention and serum-supplemented media were 18 and 17 hours, respectively, while at the twelfth passage, the generation times were 18.5 and 18 hours, respectively. Prolonged passage in the medium of the present invention did not affect antibody production as tabulated in

Table III.

Table III. Effect of prolonged passage in serum-free medium  on antibody production[a]

| Passage Number | Antibody titer (expressed as $OD_{405}/10^4$ cells in | |
| | Serum-Free Medium[b] | Serum-Supplemented Medium[c] |
| --- | --- | --- |
| 1 | 8.6 | 5.8 |
| 3 | 10.2 | 8.5 |
| 6 | 8.0 | 5.9 |
| 11 | 9.0 | 6.3 |

[a]S1-1-D4(E2) cells were serially subcultured in either serum-free medium or medium-supplemented with 10% FBS. The supernatants were periodically collected and the levels of antibodies specific to keyhole limpet hemocyanin determined by the quantitative ELISA assay.

[b]Medium of the present invention.

[c]Basal medium supplemented with 10% fetal bovine serum. The levels of keyhole limpet hemocyanin antibodies in the serum-free medium were between 8 and 10 $OD_{405}$ units per $10^4$ cells, regardless of the passage number. The levels of antibodies in the supernatants collected at various passages in serum-supplemented medium also remained relatively constant although the levels tended to be slightly lower than in the medium of the present invention.  In serum-supplemented medium, the levels of antibodies in the culture supernatants ranged from approximately 5.5 to 8.5 $OD_{405}$ units per $10^4$ cells.


### EXAMPLE III

It was noted that other mouse myeloma and hybridoma cells grew well in the media of the present invention. For example, FO, a variant of SP2/0-Ag-14, reached a density that was approximately 50% of that attained in serum. The present invention also was an excellent medium for growing MPC-11 mouse myeloma cells. In contrast P3-NS-1-Ag-4-1 mouse myeloma cells did not grow at all in the medium of the present invention. Two of the three P3-NS-1-Ag-4-1 hybridoma cell lines tested also failed to grow in the serum-free medium. However, the one cell line that did grow [N10-4-D4(F4)] reached a cell density after five days in culture that was 10-fold greater than the initial plating value and approximately 30% of that attained in basal medium supplemented with 10% fetal bovine serum. These results have been summarized in Table IV.

TABLE IV. Growth of other murine myeloma and hybridoma
cell lines in serum-free medium[a]

| Cell Line | Description | Cells/ml in | |
|---|---|---|---|
| | | Serum-Free Medium[e] | Serum-Supplemented Medium[f] |
| FO[b] | Variant of SP/20-Ag-14 Myeloma | $4.4 \times 10^5$ | $9.5 \times 10^5$ |
| MPC-11[b] | Myeloma MPC-11 | $5.2 \times 10^5$ | $7.8 \times 10^5$ |
| P3-NS-1-Ag-4-1 | Parental Myeloma NS-1 | $1.5 \times 10^4$ | $1.1 \times 10^6$ |
| N10-3-D8(E5)[c] | NS-1 hybridoma[d] | $1.4 \times 10^4$ | $2.9 \times 10^5$ |
| N10-3-C10(D6)[c] | NS-1 hybridoma[d] | $1.6 \times 10^4$ | $7.1 \times 10^5$ |
| N10-4-D4(F4)[c] | NS-1 hybridoma[d] | $2.0 \times 10^5$ | $5.9 \times 10^5$ |

[a]Cells of each type ($2 \times 10^4$/ml) were plated in 24 well
microtiter plates in serum-free medium and medium-
-supplemented with 10% fetal bovine serum.

[b]Counted four days after plating.

[c]Counted five days after plating.

[d]These hybridomas are producing antibodies to keyhole
limpet hemocyanin.

[e]Medium of the present invention.

[f]Basal medium supplemented with 10% fetal bovine serum.


## EXAMPLE IV

The medium of the present invention will also support
the growth of human lymphoblastoid cell lines. Four
cell lines (BM, AR, 8866 and 8226) all established in
culture from patients with multiple myeloma, were cultured in the medium of the present invention. Their generation times in the serum-free medium were all approximately 20 hours. In serum-supplemented basal medium they all grew with doubling times of 15-18 hours.

CLAIMS:

1. A medium for culturing cells under serum-free or low serum conditions characterised in that the medium comprises:

    A. Growth factors selected from the group consisting of:

        1. Approximately 0-100 µg/ml insulin

        2. Approximately 1-100 µg/ml transferrin

        3. Approximately 0-200 µM ethanolamine;

    B. Approximately 100-1000 µg per ml bovine serum albumin (BSA) complexed in a molar ratio ranging from 1:0.5 to 1:5 with at least one fatty acid selected from the group consisting of:

        1. arachadonic acid

        2. oleic acid

        3. linoleic acid

        4. palmitic acid

        5. stearic acid

        6. linolenic acid

        7. palmitoleic acid;

    C. Amino acids selected from the group consisting of:

        1. Approximately 0-100 mg/l L-alanine

        2. Approximately 100-1000 mg/l L-arginine

        3. Approximately 0-100 mg/l L-asparagine

        4. Approximately 0-100 mg/l L-aspartic acid

        5. Approximately 0-100 mg/l L-glycine

6.  Approximately 7.5-150  mg/l L-histidine

7.  Approximately 10-500 mg/l L-isoleucine

8.  Approximately 25-500 mg/l L-leucine

9.  Approximately 20-400 mg/l L-lysine HCl

10. Approximately 7.5-150 mg/l L-methionine

11. Approximately 0-150 mg/l L-serine

12. Approximately 10-200 mg/l L-threonine

13. Approximately 7.5-150 mg/l L-phenylalanine

14. Approximately 2.5-25 mg/l L-tryptophan

15. Approximately 10-100 mg/l L-valine

16. Approximately 0-100 mg/l L-glutamate

17. Approximately 0-200 mg/l L-proline

18. Approximately 0-100 mg/l hydroxy-L-proline

19. Approximately 10-100 mg/l tyrosine

20. Approximately 25-500 mg/l cystine

21. Approximately 100-3000 mg/l glutamine

22. Approximately 0-5.5 mg/l L-$\alpha$-amino-n-
    butyric acid

23. Approximately 0-3.9 mg/l D-glucosamine HCl

24. Approximately 0-4.2 mg/l L-taurine

25. Approximately 0-9.4 mg/l L-ornithine HCl;

D.Vitamins selected from the group consisting of:

1.  Approximately 0-5 mg/l p-amino benzoic acid

2.  Approximately 0-3 mg/l biotin

3.  Approximately 0.1-3 mg/l d-Ca-pantothenate

4.  Approximately 1.0-10.0 mg/l choline chloride

5.  Approximately 0.5-5 mg/l nicotinamide

6. Approximately 0.5-3 mg/l pyridoxine-HCl

7. Approximately 0.1-1.0 mg/l riboflavin

8. Approximately 0.5-3.0 mg/l thiamine HCl

9. Approximately 0-10.0 mg/l vitamin $B_{12}$

10. Approximately 10.0-50.0 mg/l inositol

11. Approximately 0-0.025 mg/l D-tacopherol acid succinate

12. Approximately 0-0.25 mg/l calciferol

13. Approximately 0-0.025 mg/l menadione

14. Approximately 0-0.0625 mg/l niacin

15. Approximately 0-0.25 mg/l vitamin A

16. Approximately 0.5-3.0 mg/l folic acid;

E. A balanced salt solution;

F. Trace compounds selected from the group consisting of:

1. Approximately 0-0.01 mg/l $CuSO_4.5H_2O$

2. Approximately $0-1 \times 10^{-8}$ M $FeSO_4.7H_2O$

3. Approximately 0-0.1 mg/l $ZnSO_4.7H_2O$

4. Approximately $0-1 \times 10^{-7}$ M LiCl

5. Approximately $0-2.5 \times 10^{-6}$ M $Na_2SeO_4$;

G. Coenzymes selected from the group consisting of:

1. Approximately 0-0.25 mg/l coenzyme A

2. Approximately 0-0.7 mg/l diphosphopyridine nucleotide

3. Approximately 0-1.0 mg/l flavin adenine dinucleotide

4. Approximately 0-1.0 mg/l triphosphopyridine nucleotide

5. Approximately 0-1.0 mg/l cocarboxylase

6. Approximately 0-1.0 mg/l uridine triphosphate, sodium;

H. Nucleic acid derivatives selected from the group consisting of:

1. Approximately 0-10 mg/l deoxyadenosine

2. Approximately 0-10 mg/l deoxyguanosine

3. Approximately 0-10 mg/l deoxycytidine HCl

4. Approximately 0-0.1 mg/l 5-methyl cytosine

5. Approximately 0-73 mg/l thymidine; and

I. Additional components selected from the group consisting of:

1. Approximately 0-20 mg/l lipoic acid

2. Approximately 0-1000 mg/l sodium pyruvate

3. Approximately 0-40 mg/l hypoxanthine

4. Approximately 2000 mg/l NaHCO$_3$

5. Approximately 1000-4500 mg/l glucose

6. Approximately 0-20 mg/l phenol red

7. Approximately 0-10 mg/l gluthathione

8. Approximately 0.50 mg/l ascorbic acid

9. Approximately 0-1.8 mg/l D-glucuronolactone

10. Approximately 0-1.8 mg/l sodium glucuronate

H$_2$O

11. Approximately 0-12.5 mg/l Tween 80.

2. A medium culturing cells under serum-free or low serum conditions characterised in that the medium comprises:

A.    Growth factors selected from the group consisting of:

1. Approximately 5 µg/ml insulin

2. Approximately 50 µg/ml transferrin

3. Approximately 20 µM ethanolamine;

B. Approximately 100-1000 µg/ml of Bovine serum albumin (BSA) complexed in a 1:1 molar ratio with each of the following fatty acids:

1. arachadonic acid

2. oleic acid

3. linoleic acid

4. palmitic acid

5. stearic acid

6. linolenic acid

7. palmitoleic acid;

C. Amino acids selected from the group consisting of:

1. Approximately 10 mg/l L-alanine

2. Approximately 200 mg/l L-arginine

3. Approximately 50 mg/l L-asparagine

4. Approximately 20 mg/l L-aspartic acid

5. Approximately 10 mg/l L-glycine

6. Approximately 15 mg/l L-histidine

7. Approximately 50 mg/l L-isoleucine

8. Approximately 50 mg/l L-leucine

9. Approximately 40 mg/l L-lysine HCl

10. Approximately 15 mg/l L-methionine

11. Approximately 30 mg/l L-serine

12. Approximately 20 mg/l L-threonine

13. Approximately 15 mg/l L-phenylalanine

14. Approximately 5 mg/l L-tryptophan

15. Approximately 20 mg/l L-valine

16. Approximately 20 mg/l L-glutamate

17. Approximately 20 mg/l L-proline

18. Approximately 20 mg/l hydroxy-L-proline

19. Approximately 20 mg/l tyrosine

20. Approximately 50 mg/l cystine

21. Approximately 300 mg/l glutamine

22. Approximately 0.55 mg/l L-α-amino-n-butyric acid

23. Approximately 0.39 mg/l D-glucosamine HCl

24. Approximately 0.42 mg/l L-taurine

25. Approximately 0.94 mg/l L-ornithine HCl;

D. Vitamins selected from the group consisting of:

1. Approximately 1 mg/l p-aminobenzoic acid

2. Approximately 0.2 mg/l biotin

3. Approximately 0.25 mg/l d-Ca-pantothenate

4. Approximately 3 mg/l choline chloride

5. Approximately 1 mg/l nicotinamide

6. Approximately 1 mg/l pyridoxine-HCl

7. Approximately 0.2 mg/l riboflavin

8. Approximately 1 mg/l thiamine HCl

9. Approximately 1.0 mg/l vitamin $B_{12}$

10. Approximately 35 mg/l inositol

11. Approximately 0.0025 mg/l D-tacopherol acid succinate

12. Approximately 0.025 mg/l calciferol

13. Approximately 0.0025 mg/l menadione

14. Approximately 0.00625 mg/l niacin

15. Approximately 0.025 mg/l vitamin A

16. Approximately 1 mg/l folic acid;

E. A balanced salt solution;

F. Trace compounds selected from the group consisting of:

1. Approximately 0.00125 mg/l $CuSO_4 \cdot 5H_2O$

2. Approximately $10^{-10}$M $FeSO_4 \cdot 7H_2O$

3. Approximately 0.028 mg/l $ZnSO_4 \cdot 7H_2O$

4. Approximately $10^{-9}$M LiCl

5. Approximately $2.5 \times 10^{-8}$M $Na_2SeO_4$;

G. Coenzymes selected from the group consisting of:

1. Approximately 0.025 mg/l coenzyme A

2. Approximately 0.07 mg/l diphosphorpyridine nucleotide

3. Approximately 0.1 mg/l flavin adenine dinucleotide

4. Approximately 0.1 mg/l triphosphopyridine nucleotide

5. Approximately 0.1 mg/l cocarboxylase

6. Approximately 0.1 mg/l uridine triphosphate sodium;

H. Nucleic acid derivatives selected from the group consisting of:

1. Approximately 1 mg/l deoxyadenosine

2. Approximately 1 mg/l deoxyguanosine

3. Approximately 1 mg/l deoxycytidine HCl

4. Approximately 0.02 mg/l 5-methyl cytosine

5. Approximately 1 mg/l thymidine; and

I. Additional components selected from the group consisting of:

1. Approximately 0.2 mg/l lipoic acid

2. Approximately 110 mg/l sodium pyruvate

3. Approximately 4 mg/l hypoxanthine

4. Approximately 200 mg/l $NaHCO_3$ .

5. Approximately 2000 mg/l glucose

6. Approximately 5 mg/l phenol red

7. Approximately 1 mg/l gluthathione

8. Approximately 5 mg/l ascorbic acid

9. Approximately 0.18 mg/l D-glucuronolactone

10. Approximately 0.18 mg/l sodium glucuronate $H_2O$

11. Approximately 1.25 mg/l Tween 80.

3. A culture medium according to claim 1 characterised in that the bovine serum albumin is complexed in an approximately 1:1 molar ratio with one or more of the fatty acids.

4.    A culture medium according to claim 1 or claim 3 characterised in that at least some palmitic acid and stearic acid complexed with bovine serum albumin is present.

5.    A culture medium according to any preceding claim characterised in that the balanced salt solution comprises Earle's salts

(a.    approximately 100 mg/l $Ca(NO_3)_2.4H_2O$

b.    approximately 400 mg/l KCl

c.    approximately 100 mg/l $MgSO_4.7H_2O$

d.    approximately 6000 mg/l NaCl

e.    approximately 800 mg/l $Na_2HPO_4$).

6.    A culture medium according to any of claims 1 to 4 characterised in that the balanced salt solution comprises Hank's balanced salt solution.

7.    A culture medium according to any preceding claim characterised in that the medium further comprises N'--2-hydroxyethyl piperazine-N-ethane-sulphonic acid as a buffer system either substituted for the carbonate--bicarbonate buffer described in claims 1 or 2 or as a secondary buffer in combination with the carbonate--bicarbonate described in claims 1 and 2.

8.    The use of a culture medium according to any preceding claim for culturing SP2/0-Ag-14 myeloma cells or SP2/0-Ag-14 hybridoma cells.

9.    The use of a culture medium according to any of claims 1 to 7 for culturing P3-NS-1-Ag-4-1 hybridoma cells, FO mouse myeloma and hybridoma cells, human lymphoblastoid cells, MPC-11 myeloma and hybridoma cells, cell lines originating from the immune system, or normal cells of the immune system cultured either as individually purified cell populations or as mixture of cells.

10.    The use of a culture medium according to any of claims 1 to 7 with serum.

11.    The use of the basal medium of claim 1 or claim 2 (all of the components except insulin, transferrin, ethanolamine, and fatty acid linked BSA) for culturing any cells originating from the immune system with any serum, from any species at any concentration.